# EUROPEAN PATENT SPECIFICATION

(11) **EP 3 628 281 B1**
(45) Date of publication and mention of the grant of the patent: **30.07.2025**
(21) Application number: 18196744.9
(22) Date of filing: 26.09.2018
(51) Int. Cl.: A61F 9/008, A61B 18/26

(54) **DEVICE FOR PLASMA TREATMENT OF BIOLOGICAL TISSUE**
VORRICHTUNG ZUR PLASMABEHANDLUNG VON BIOLOGISCHEM GEWEBE
DISPOSITIF D'UN TRAITEMENT AU PLASMA DE TISSU BIOLOGIQUE

(43) Date of publication of application: 01.04.2020
(73) Proprietor: A.R.C. Laser GmbH, 90411 Nürnberg (DE)
(72) Inventor: THYZEL, Reinhardt, 90542 Eckental (DE)
(74) Representative: Meissner Bolte Nürnberg

(56) References cited:
- US-A1- 2006 200 113
- US-A1- 2007 161 981
- US-A1- 2014 012 186

## Description

The present invention is directed to a device for the treatment of biological tissue, in particular for in vivo plasma treatment of biological tissue, such as for example the trabecular meshwork of the eye of for example a human being.

The trabecular meshwork of the human eye is a kind of porous meshwork and is part of a drainage system for draining eye liquid (*humor aqueous*) generated by the ciliary bodies. In the healthy eye, the eye liquid flows from the posterior eye chamber through the pupil and the anterior eye chamber and is drained over the trabecular meshwork and the Schlemm's canal arranged downstream the trabecular meshwork.

Disturbance of the drainage of the eye liquid, for example through blockage or congestion of the trabecular meshwork, may lead to an increased intraocular pressure (ocular hypertension), which in turn may cause Glaucoma. Removing the blockage or congestion of the drainage system, in particular the trabecular meshwork, is one possibility to eliminate ocular hypertension, and the occurrence of Glaucoma which would otherwise be caused by continued blockage or congestion of the trabecular meshwork.

Thus, there is a need for devices suitable for in vivo tissue treatment, and appropriate for eliminating blockage in the drainage system of the eye, in particular in the trabecular meshwork of the eye.

The object of the present invention is thus to provide a respective device and operational method for tissue treatment, in particular a device that is suitable for treating the drainage system, in particular trabecular meshwork, of the eye, for example in connection with avoiding Glaucoma caused by disturbances in the draining of the eye liquid in the region of the trabecular meshwork of the eye.

This object is solved in accordance with the invention by the annexed independent claims. Embodiments of the invention result from the dependent claims and the description below.

In embodiments, a device for the treatment of biological tissue, in particular for in vivo plasma treatment of biological tissue, of for example a human or animal organism is provided. The biological tissue may be the trabecular meshwork of the human eye. The treatment of the trabecular meshwork may involve blockage removal within the trabecular meshwork for the avoidance of or removal of ocular hypertension. Treating the trabecular meshwork with a plasma generated by the device described herein below leads to increased penetrability and permeability of the trabecular meshwork for eye liquid, in turn leading to improved discharge capabilities for eye liquid, such that ocular hypertension may be removed or avoided. Ocular hypertension is one of the most important risk factors for the development of Glaucoma.

In embodiments, the device may comprise a hollow needle-like tubular section with a distal end for applying a light-induced plasma to the tissue. For example, the distal end may be positioned on or on the tissue to be treated, such that a light-induced plasma discharged from the device may impinge the tissue. The needle-like tubular section may be used as a needle for penetrating, for example, surrounding tissue and the tissue to be treated as far as necessary. For example, the needle-like section may be inserted into the tissue with the distal end ahead such that the distal end can be placed at the tissue to be treated with plasma discharged from the distal end of the device.

In embodiments, the tubular section may comprise subsequent to (or: adjacent to) the distal end an inner plasma chamber. The inner plasma chamber may be configured for generating therein, or at least for inducing (or: seeding) therein, a light-induced plasma.

According to embodiments, the distal end comprises a distal, central axial aperture (in particular: a central axial hole), wherein the plasma chamber is communicatively coupled with the distal, central axial aperture. Further, the plasma chamber adjoins, within the tubular section, a light injection section for injecting light, in particular light pulses, into the plasma chamber for plasma generation (or: formation), to be discharged at least in part, for example as a plasma cloud, out of the plasma chamber for plasma treatment of the tissue.

The aperture is configured for and adapted to enabling light-induced plasma, and possibly shockwaves accompanying the plasma, to propagate through the aperture to the outside. By providing the plasma chamber in connection with the aperture, the plasma generation, in particular the processes of plasma generation, may be confined to the plasma chamber, such that only discharged plasma may interact with the tissue. Using the plasma and associated effects, such as shockwaves, propagating through the aperture may be advantageous for gentle tissue treatment.

In particular, the light injection section, the plasma chamber and the aperture may be arranged successively along the longitudinal axis of the tubular section such that a light pulse, in particular a laser light pulse, may be injected into the plasma chamber from the light injection section. Once the irradiation threshold for plasma formation within the plasma chamber is exceeded, a plasma is generated, by optical breakdown, e.g. by multiphoton avalanche ionization.

The plasma generated or induced within the plasma chamber, for example by nanosecond laser-pulses, evolves and propagates within the plasma chamber, in particular towards the incoming pulse, for example opposite to the light input direction. Due the fact that the plasma chamber defines a confined volume that is communicatively coupled with the environment via the aperture, the plasma can propagate and spread out of the plasma chamber, i.e. discharge out of the plasma chamber. The portion of the plasma propagating and spreading out of the plasma chamber can, at least in part, be used for tissue treatment. Interactions between the plasma and the tissue result in plasma-mediated distortions, such as ablation and disruption, which, if applied to the trabecular meshwork for example result in improved permeability or penetrability for eye fluid. Propagation of the plasma within and out of the plasma chamber may be accompanied by shockwaves generated in connection with an optical breakdown causing the plasma formation.

The primary purpose of the device resides in the plasma-induced (or: plasma-mediated) treatment of tissue. As noted above, the generation of the plasma, e.g. by optical breakdown, in particular laser-induced breakdown, may be accompanied by shockwave formation which may affect plasma propagation, and, beyond that, contribute as such to the tissue treatment. In other words, the plasma-mediated effects for treating the tissue are the intended primary effects for tissue treatment, and secondary effects for example induced by shockwave formation, may be present and may contribute to the overall impact of the treatment.

By providing the plasma chamber, laser light emitted from the light injection section can be confined at least to a large extent to the plasma chamber. A portion of the laser light may escape from the plasma chamber prior to reaching the irradiance threshold for plasma formation.

As soon as the irradiance threshold is reached, the laser energy is absorbed substantially completely for plasma generation, wherein plasma formation in the plasma chamber prevents the laser light from escaping the chamber.

The portion of the light that is able to escape the chamber prior to and/or after plasma formation, has comparatively low energy and low energy density, with only moderate, low or even negligible impact on the tissue. In particular, the effects of the laser light as such on the tissue may, with regard to tissue treatment, may (substantially) be neglected if compared to plasma-mediated tissue treatment.

The device as described herein, providing a plasma discharged from the plasma chamber for tissue treatment, is particularly suitable for treating the trabecular meshwork of the eye. In particular, it has been shown that the plasma-mediated treatment of the trabecular meshwork by using the device as described herein is effective for removing blockage of the trabecular meshwork and for improving permeability of the trabecular meshwork for eye liquid, for example by generating one or more holes or perforating at lease sections of the trabecular meshwork, to thereby reduce the risk of Glaucoma caused by ocular hypertension.

In embodiments, the aperture, the plasma chamber, and the light injection section are arranged coaxially with each other and, in particular, with the longitudinal axis of the tubular section. In particular, the light injection section may face (or: be oriented towards) the distal, central axial aperture, and the normal of the aperture as well as the optical axis of the light injection section may coincide with the longitudinal central axis of the plasma chamber which in turn may coincide with the longitudinal axis of the tubular section. Such an arrangement in particular may provide expedient plasma formation properties, suitable for discharging plasma out of the plasma chamber for tissue treatment. Further, such a design enables simple and compact construction of the device, in particular the tubular section.

In embodiments, the cross section of the aperture measured perpendicular to the longitudinal axis of the plasma chamber or of the tubular section is in the range from 0.05 mm to 0.5 mm, preferably 0.05 mm to 0.3 mm. Such cross sections in particular have been proven advantageous for generating and discharging a plasma for treating the trabecular meshwork for the purpose of enhancing the permeability and penetrability for eye liquid.

In embodiments, the distal end of the tubular section is dome-shaped. Further, the distal end may have an outer rounded tip, wherein a ratio of the outer diameter of the tubular section and the bending radius of the tip end may be at in the range of about 2:1.

In embodiments, the distal end may comprise an inner, axial face that is oriented towards the plasma chamber. The inner axial face may delimit the plasma chamber in axial direction towards the aperture. Further, the axial face may surround the axial aperture, and may, for example, have an annular ring shape.

In embodiments, the axial face may, in axial direction towards the aperture, be conically tapered. In particular, the axial face may form a transitional zone for diameter reduction between the inner circumferential wall of the plasma chamber and the distal aperture. The tapering of the axial face may support plasma formation and/or plasma discharge through the aperture.

Further, the tapering of the axial face may be selected to obtain a particular (or: a desired) shape of the discharged plasma.

In embodiments, the tapering of the axial face may be such that an opening angle of the conically tapered axial face is in the range from 115 to 120 degrees, preferably 118 degrees. Such opening angles in particular have been proven effective for treating the trabecular meshwork for the purpose of improved penetrability and permeability for eye liquid.

In embodiments, the outer diameter of the tubular section, in particular or at least in the region of the plasma chamber, may be in the range between 0.6 mm and 1.0 mm, preferably 0.8 mm. Further, the inner diameter of the tubular section, in particular or at least in the region of the plasma chamber, may be in the range between 0.5 to 0.7 mm, preferably 0.6 mm. Further, the outer and inner diameter of the tubular section may be constant over the whole length of the tubular section, in particular at least over the whole length of the plasma chamber except of course in the region of the tapered axial face where the inner diameter is reduced to the cross section of the aperture.

According to further embodiments, a ratio between the outer diameter and the inner diameter may be in the range of about 4:3.

Such inner and outer dimensions of the tubular section and/or the plasma chamber or parts thereof have been proven advantageous for treating the trabecular meshwork to improve penetrability and permeability for eye liquid.

In embodiments, the wall thickness of the tubular section at the distal end measured parallel to the longitudinal axis of the tubular section is in the range from 0.18 mm to 0.22 mm, preferably 0.2 mm. By this, the distal end may provide sufficient mechanical stability and may provide sufficient material strength to act as a laser absorption target for absorbing laser light.

In embodiments, the axial length of the plasma chamber measured parallel to the longitudinal central axis of the tubular section is at least as large as the inner diameter of the tubular section.

Further, in embodiments the axial length of the plasma chamber may be, at least by a factor of 1.5, in particular of about 1.66, larger than the inner diameter of the tubular section.

In further embodiments, the axial length of the plasma chamber may be, in absolute terms, in the range from 0.5 mm to 1.2 mm, in particular 0.9 mm to 1.1 mm.

Such plasma chamber dimensions have proven advantageous for generating a plasma discharge from the plasma chamber suitable for treating biological tissue, in particular the trabecular meshwork in connection with enhancing penetrability and permeability for eye liquid.

In embodiments, the light injection section has a numerical aperture (in air) in the range from 0.2 to 0.24, in particular 0.22.

According to further embodiments, the light injection section may include as an axial end face facing the plasma chamber, a light exit face, in particular a laser light exit face, of a light guide, such as for example an optical fiber, and/or of a fiber optic, for example associated with a light guide or optical fiber. The light guide may be guided in and arranged coaxially within the tubular section, the plasma chamber and/or the aperture.

In embodiments, the axial end face, in particular the light exit surface, may, in embodiments, be oriented towards the aperture. The light guide may be disposed within the tubular section such that, in particular in the region of the axial end face, the optical axis of the light guide is coaxial with the tubular section, in particular the longitudinal axis of the tubular section, the plasma chamber and/or the aperture.

Further, the light guide, in particular the light exit face, the light exit face, and/or the fiber optic may have a numerical aperture (in air) in the range from 0.2 to 0.24, in particular 0.22. In embodiments, a numerical aperture (in air) of 0.2 to 0.24 may be implemented in connection with an opening angle for emitted light in the range of about 25 degrees.

The suggested configuration of the light injection section and light guide, in particular their arrangement and the numerical aperture, has been proven advantageous in connection with generating the plasma within the plasma chamber and discharging at least a part of the plasma out of the plasma chamber.

In embodiments, the light guide may be arranged adjacent to the plasma chamber, in particular immediately adjacent to the plasma chamber, such that the light exit surface of the light guide faces the inner axial face. The light guide may be configured and arranged such that at least a portion of the light exiting the light exit surface impinges on the inner axial face, to generate or at least to partially contribute to inducing, within the plasma chamber, a plasma by optical breakdown in which volumetric material associated with the inner axial face may act as a light absorption target.

In embodiments, the device may further comprise a tubular extension, in particular a hollow tubular extension, preferably arranged immediately adjacent to the end of the tubular section averted from the distal end. The tubular extension may be arranged coaxially with the tubular section. The tubular extension may have an outer diameter that is larger than the outer diameter of the tubular section. In embodiments, the inner diameter of the tubular extension may substantially correspond to the outer diameter of the tubular section.

In embodiments, the tubular extension may be configured for being inserted and mounted within a recess provided in a mounting head. The mounting head may be configured for being mounted to a handpiece that is suitable for manual handling of the device.

In embodiments, the device may further comprise the handpiece. In such embodiments, the mounting head may be fastened to one end of the handpiece.

Further, a flexible duct for guiding therein the light guide, for example an optical fiber associated with the light injection section, may be attached to the other end of the handpiece. The flexible duct may be disposed at the end averted from the one end to which the mounting head is fastened.

In embodiments, the light guide, in particular the optical fiber, may be fastened within or at the hollow tubular section, the tubular extension, the handpiece and/or the flexible duct by means of at least one adhesive bond. By this, adequate fixation for reliable and uniform plasma generation and formation may be obtained.

In embodiments, the tubular section, including the distal end, and optionally also the tubular extension may be made from titanium or a titanium alloy. The handpiece and/or the mounting head may be made from a plastic material.

In embodiments, the device may further comprise a light source, such as a laser light source. The light source may be configured for and adapted to supplying, via the light guide, light, e.g. laser light, to the light injection section, in particular for emission into the plasma chamber for plasma formation, by suitable optical coupling with the light guide.

The laser light may be a pulsed laser light. Further, the laser light source may be a Q-switched laser, e.g. a Q-switched Nd:YAG laser. The light source may be configured and adapted to generate laser pulses having a pulse width of 8 ns ± 3 ns, and/or a pulse energy in the range from 6 mJ to 10 mJ. Such operational parameters have been proven advantageous for generating a plasma suitable for treating the trabecular meshwork as described in further detail further above.

Embodiments of the invention relate to the use of a device according to any embodiment described herein for treatment of Glaucoma by removing blockage of the trabecular meshwork of the human or animal eye by applying a light-induced plasma generated within or induced at least in part within the plasma chamber of the device, and discharged out of the plasma chamber for treatment of the trabecular meshwork. Removing blockage may include generating or creating one or more holes and/or perforating the trabecular meshwork at least in part, e.g. in pre-defined sections.

In this connection, the expression "treatment of Glaucoma" shall be understood as the process of removing or avoiding the adverse effects caused by ocular hypertension in turn caused by impaired penetrability and permeability of the trabecular meshwork for eye liquid. The use of a plasma, generated for example by a device according to any embodiment described herein, for the treatment of the trabecular meshwork has proven as an efficient and comparatively gentle way to improve penetrability and permeability for eye liquid.

Embodiments of the invention may relate to a method, in particular an automated method, e.g. a computer-implemented method, of operating a device according to any embodiment described herein, wherein the method may comprise generating, by a light source, in particular a laser light source, such as a Q-switched laser, at least one, preferably a plurality of, light pulses, in particular laser light pulses, and feeding the at least one light pulse by means of a light guide (in particular: through the light guide) to the light injection section to generate a plasma or at least to induce or seed a light-induced plasma within the plasma chamber such that at least a part of the plasma, in particular in form of a plasma cloud, is discharged out of the plasma chamber for tissue treatment, in particular for tissue treatment, for the treatment of Glaucoma.

In particular, the device may be operated such that a plasma is generated or seeded within the plasma chamber, and such that the generated or seeded plasma propagates and is discharged out of the plasma chamber for tissue treatment.

In embodiments of the method, generating the at least one light pulse may comprise generating a laser pulse, in particular a nanosecond laser pulse. The laser pulse may be generated to have a pulse width of 8 ns ± 3 ns, and/or a pulse energy in the range from 6 mJ to 10 mJ.

Exemplary embodiments of the invention will now be described in connection with the annexed figures in which:
- FIG. 1: shows a plan view of a handheld device for in vivo tissue treatment in accordance with the invention;
- FIG. 2: shows plan view of a handpiece of the handheld device in a partly exploded view;
- FIG. 3: shows an enlarged view of a tool tip of the handpiece;
- FIG. 4: shows a perspective view of a tubular tip of the handpiece of FIG. 3;
- FIG. 5: shows a plan view of the tubular tip of FIG. 4;
- FIG. 6: shows a cross section along the longitudinal axis L of the tubular tip in FIG. 5;
- FIG. 7: shows an enlarged view of the section of left-hand tip end of the tubular tip, marked in FIG. 6; and
- FIG. 8: also shows the enlarged view of the tip end of the tubular tip, marked in FIG. 6.

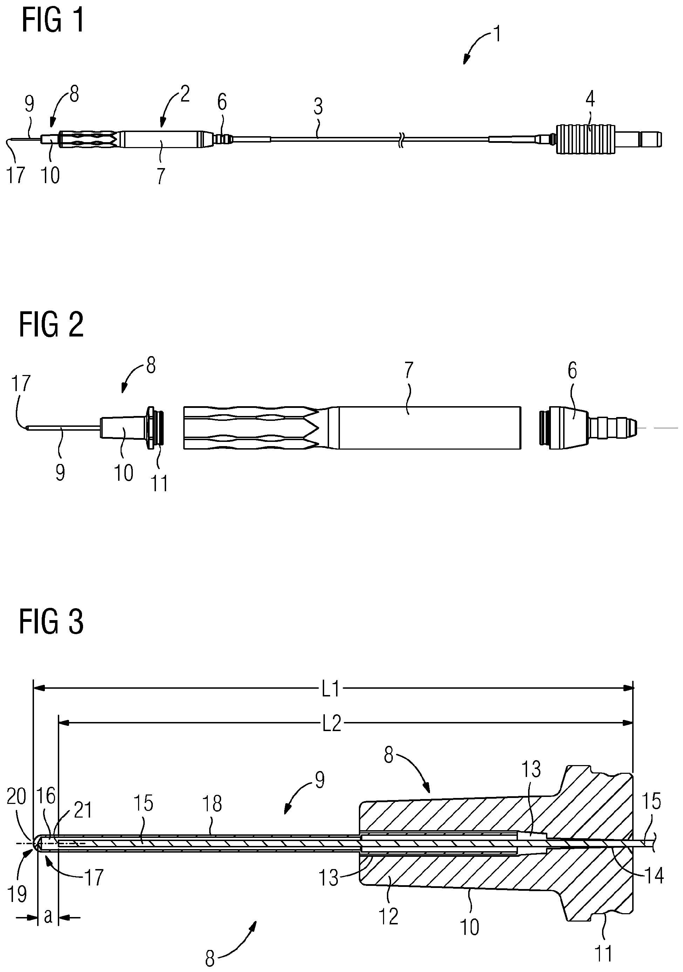

Figure 1 shows a plan view of a handheld device 1 for use tissue treatment in accordance with the invention, for example for use in in vivo tissue treatment.

The handheld device 1 comprises a handpiece 2 for manual handling. One end of the handpiece 2 is connected with a flexible (in particular: bendable) light supply line 3 which, at the side averted from the handpiece 2, is connected with an connector piece 4 for connecting the handheld device 1 with a light generator or light source (not shown), for example a laser light generator. The laser light generator may comprise a Q-switched Nd:YAG laser adapted to generate laser pulses with a pulse width of 8ns ± 3 ns and a pulse energy in the range from 6 mJ to 10 mJ.

As can be seen in greater detail from FIG. 2, a routing element 6 may be provided on a shaft 7 of the handpiece 2, wherein the routing element 6 is configured for avoiding undue bending and nocuous bending radii of the supply line 3 in regions near the end of the shaft 7.

At the side averted from the light supply line 2 and routing element 6, a tool tip 8 is attached (in particular: mounted, for example by adhesive bonding) to the shaft 7 of the handpiece 2. The tool tip 8 comprises, as is also apparent from FIG. 3, a tubular tip 9 comprising a hollow needle-like tubular section, and a mounting head 10.

The mounting head 10 comprises a (male) coupler section 11 (see also FIG. 3) configured for being coupled with one end of the shaft 7 (e.g. a female counterpart), for example by a mechanical bond, and adhesive where appropriate. The mounting head 10 further includes a retainer section 12 with a recess 13 opening at one end of the mounting head 10.

The recess 13 is designed such that the tubular tip 9 can be attached to (in particular: mounted to) the mounting head 10 by inserting one end of the tubular tip 9 into the recess 13. The tubular tip 9 may be secured within the recess 13 by adhesive bonding.

A central bore 14 is provided in the mounting head 10 such that the mounting head includes a central passage for passing through an optical guide, in particular an optical light guide, such as an optical fiber 15, which is shown in FIG. 3.

As will be apparent from the combination of FIG. 1 to FIG. 3, the optical fiber 15 is guided from the tubular tip 9 through the supply line 3 to the connector piece 4. By connecting the connector piece 4 with an appropriate light source (not shown), in particular a laser light source, light, for example in the form of laser light pulses described above, can be guided through the optical fiber from which the pulses may be injected into a chamber 16 provided within the hollow tubular tip 9 at a tip end 17 thereof. Adhesive bonding may be used to attach and fix the optical fiber 15 at components of the device.

The chamber 16 is formed as a hollow space or cavity between the distal end of the optical fiber 15 and an inner distal end section of the tip end 17.

The axial length of the chamber 16 measured parallel to the longitudinal axis L of the tubular tip 9, may for example be in the range from 0.9 mm to 1.2 mm. The axial length of the chamber 16 may for example be defined by the position of the distal end of the optical fiber 15. In particular, during assembly, the optical fiber 15 may be inserted into the tubular tip 9 until a desired distance between the distal end of the optical fiber 15 and the inner distal end section of the tip end 17 is obtained. The distance may be representative of the axial length of the chamber 16.

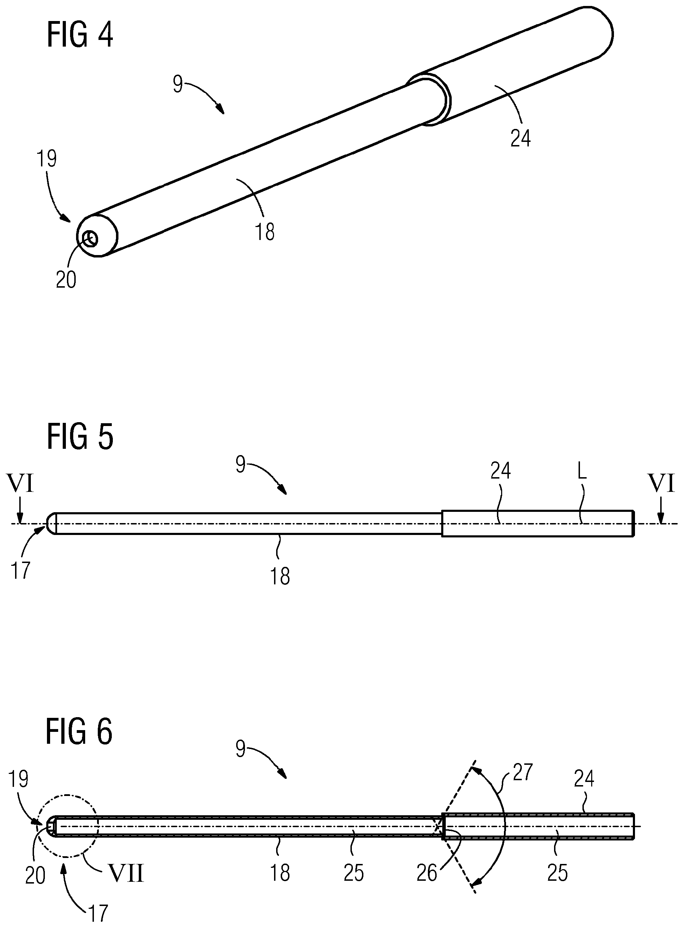

The structure and operation of the hollow tubular tip 9 for the in vivo treatment of biological tissue, such as the trabecular meshwork of the human eye for avoiding Glaucoma, will be described in detail in the following sections, and with reference to FIG. 4 through FIG. 8.

Figure 4 shows a perspective view of the tubular tip 9. The tubular tip 9 comprises a hollow needle-like (in particular: acicular) tubular section 18 with a distal end 19 suitable for insertion into the tissue to be treated, such as the trabecular meshwork.

The outer diameter of the optical fiber 15 substantially corresponds to the inner diameter of the tubular section 18, wherein the inner diameter of the tubular section may be slightly larger than the diameter of the optical fiber 15 in order to facilitate insertion of the fiber into the tubular tip 9. Such dimensions are advantageous in particular for disposing the optical fiber 15 coaxially with the tubular section 18 and, other parts of the tubular tip 9, such as the chamber 16.

As already mentioned, the optical fiber 15 may be fixed within the tubular section 18, in particular the tubular tip 9, by adhesive bonding, wherein the adhesive bonding may be applied such that a liquid- and/or gas- or air-tight seal between the outer surface or an outer circumference of the optical fiber 15 and the inner surface of one or more corresponding components surrounding the optical fiber 15, such as the tubular section 18, the supply line 3 etc., is obtained, for example at one or more particular locations in lengthwise direction of the optical fiber 15. By this, the optical fiber 15 can be firmly held within the device components, and the channel in which the optical fiber is guided can be protected against environmental influences, wherein undesired substances, such as liquids, can be prevented from infiltrating the channel.

Such adhesive bonding and similar fixing techniques for the optical fiber 15 are possible because the plasma-induced treatment of tissue enabled by the proposed device may be carried out without the need to rinse or drain the tissue to be treated, or to extract tissue debris. In particular, the tissue treatment by a plasma generated within the chamber 16 and discharged therefrom, may be carried out without supplying or removing auxiliary or additional liquids or substances through the tubular section 18, in particular the tubular tip 9. Specifically, the device may be designed without additional or auxiliary inner channels for rinsing, draining or extracting liquid(s), debris or other substances from the treatment area, which is advantageous with regard to simplified design and manufacture, and, beyond that, contributes to simplified operation. For the reason that the operation of the device results in a plasma generated (or: induced) in the chamber 16, the chamber 16 is referred to herein also as plasma chamber.

Directly adjacent to the distal end 19, the tubular section 18 includes the inner plasma chamber 16, configured for generating therein a light-induced, in particular laser-light induced, plasma, for example by optical breakdown.

The plasma may be generated for example by emitting a laser pulse (not shown) into the plasma chamber via the optical fiber 15, which may have a numerical aperture of 0.22.

As soon as the energy of the laser pulse, for example with regard to the time-profile of the laser pulse, exceeds an irradiance threshold, non-linear absorption processes may lead to laser-induced breakdown and plasma formation (or: generation). The plasma evolves and propagates within the plasma chamber, and is, based on the design suggested herein, at least in part discharged from the plasma chamber 16, which will be described in greater detail below. The plasma formation may be accompanied by a shockwave that may also be discharged from the plasma chamber 16. Regarding the impact of the shockwave, reference to the discussion further above is made.

As may be inferred from FIG. 3, FIG. 7, and FIG. 8, the inner plasma chamber 16 is located adjacent to the distal end 19 of the tubular section 18, and is communicatively, in particular fluidly, coupled with a distal, central axial aperture 20 in the distal end 19 of the tubular section 18. In the assembled state, the aperture 20, the tubular section 18, the plasma chamber 16 and the optical fiber 15, as far as guided within the tubular section in the region of and near the plasma chamber 16, are arranged coaxially with one another.

The plasma chamber 16 defines a contiguous inner space or compartment, and is arranged immediately adjacent to a light injection section 21 (see FIG. 3, FIG. 7, FIG. 8) at the distal end of the optical fiber 15. The plasma chamber 16 may be rotationally symmetric with regard to the longitudinal axis L, and may have for example a tubular, in particular cylindrical shape.

The light injection section 21 (in particular: light injection area) in the shown example embodiment is located fully within the tubular section at a pre-defined distance from the central axial aperture 20 or distal inner wall of the distal end 19.

The light injection section 21 in the example embodiment shown in the figures includes as an axial end face facing the plasma chamber 16, a light exit face of the optical fiber 15. The optical fiber 15 is disposed within the tubular section such that the optical axis of the optical fiber coincides with the longitudinal axis L, i.e. is coaxial with the longitudinal axis L of the tubular section 18, in particular the plasma chamber 16.

In particular, the plasma chamber 16, the central axial aperture 20, and the light injection section 21 are configured such that a light-induced plasma generated within the plasma chamber 16 by light injection, in particular laser light injection, into the plasma chamber 16 at least in part is discharged from the plasma chamber 16 through the central axial aperture 20.

During ordinary operation, the part of the plasma discharged from the plasma chamber 16, impinges on the tissue located near or in front of the aperture 20, and the tissue may be modified (or: treated) by plasma absorption. Plasma absorption may be accompanied by shockwave absorption resulting from shockwaves generated in connection with plasma formation. Applied to the trabecular meshwork of the eye, the tissue modification by plasma absorption as suggested herein in particular is such that penetrability and permeability of the trabecular meshwork may be improved such that ocular hypertension as far as caused by blockage of the trabecular meshwork may be eliminated.

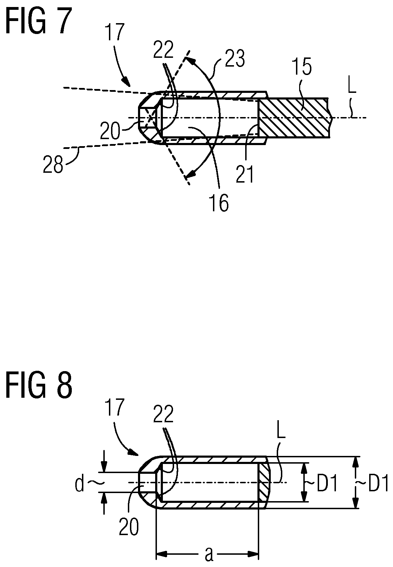

The axial length a of the plasma chamber 16 (see FIG. 8) measured along the central longitudinal axis L of the tubular section 18 is at least as large as the inner diameter of the tubular section 18. For example, the axial length a of the plasma chamber 16 may be larger than the inner diameter D1 of the tubular section 18 at least by a factor of 1.5, for example by a factor of about 1.66.

According to exemplary embodiments, the axial length of the plasma chamber may be 1 mm ± 0.2 mm, in cases where the inner diameter D1 is in the range from 0.3 mm to 0.6 mm. Further, the given dimensions may be used in connection with a laser source that is adapted to generate laser pulses having a pulse length of 8 ns ± 3 ns and a pulse energy of 6 mJ to 10 mJ for plasma generation. In such exemplary embodiments, plasma generated within the plasma chamber and discharged therefrom may be used for treating the trabecular meshwork as described herein.

As may be inferred from FIG. 4 through FIG. 8, the distal end 19 of the tubular section 18 may be dome-shaped, and may have an outer rounded tip. Such a structure is advantageous with regard to discharging a plasma cloud from the plasma generated within the plasma chamber 16 outwards, and at the same time provides satisfactory properties with regard to tissue insertion penetration, and interaction.

The ratio of the outer diameter D2 of the tubular section and the bending radius of the tip end 17 may for example be in the range of about 2:1.

Regarding absolute geometrical dimensions, in exemplary embodiments, the outer diameter D2 of the tubular section may be in the range of about 0.8 mm. Further, the inner radius D1 may be in the range of about 0.6 mm. Yet further, the bending radius of the tip end 17 or the distal end 19 may be in the range of about 0.4 mm.

The central axial aperture 20 may be configured such that the inner diameter D1 of the tubular section 18 is 2 to 12 fold larger than the cross section of the central axial aperture 20 measured perpendicular to the longitudinal axis L.

In exemplary embodiments, and expressed in absolute terms, the diameter of the central axial aperture 20 may lie in the range from 0.05 mm to 0.5 mm, for example 0.3 mm.

The geometrical dimensions as described beforehand have proven advantageous for discharging the plasma, in particular an appropriate portion of the plasma, out of the plasma chamber 16 such that appropriate treatment of tissue, for example the trabecular meshwork, may be obtained.

As described above, the discharge of a plasma cloud through the central axial aperture 20 and/or the plasma formation within the plasma chamber, may be accompanied by an acoustic shockwave. The shockwave may pass through the central axial aperture 20, in particular subsequent to discharging plasma out of the plasma chamber, wherein the shockwave may contribute to tissue modification as described above.

The interaction of the plasma and the tissue, such as the particular trabecular meshwork, and the interaction of the acoustic wave with the trabecular meshwork increases, for example in connection with using laser pulses as defined further above, the porosity of the trabecular meshwork, i.e. the penetrability and permeability of the trabecular meshwork for eye liquid. By this, ocular hypertension generated by blocked and congested areas of the trabecular meshwork may be eliminated, thereby reducing the risk for Glaucoma.

As may be inferred from FIG. 7 and FIG. 8, the distal end 19 comprises an inner, axial face 22 that is oriented towards the plasma chamber 16. The inner axial face 22 in the embodiment shown in connection with the figures surrounds the inner aperture area of the central axial aperture 20, and has an annular ring shape.

The inner axial face 22 may act as a target for a part of the laser light 28 emitted from the light injection section 21 and the optical fiber 15, wherein the laser light 28 is emitted under a particular opening angle, which is associated with a predetermined numerical aperture, which may be in the range of about 0.22.

Specifically, and as may be inferred from FIG. 3, the optical fiber 15 is arranged adjacent to the plasma chamber 16 such that the light exit surface, i.e. the light injection section 21, faces the inner axial face 22. By this, light emitted from or exiting the light exit surface at least in part may be directed towards and impinge on the inner axial face 22, which may contribute to plasma formation within the plasma chamber 16.

The plasma within the plasma chamber 16 may be generated by laser absorption associated with an optical breakdown, wherein volumetric material associated with the inner axial face 22, and/or of the tubular section in the region of the inner axial face 22, may act at least in part as a light absorption seed for plasma formation.

Regarding the inner axial face 22, the plasma generation may be improved if, as with the example embodiment shown in FIG. 7, the axial face is conically tapered in axial direction towards the aperture. Further, it is advantageous for plasma generation that the opening angle 23 of the conically tapered axial face 22 is in the range from 115 to 120 degrees, for example 118 degrees.

As may be inferred from FIG. 4 to FIG. 6, a hollow tubular extension 24 may be provided in addition to the tubular section 18. The hollow tubular extension 24 is arranged immediately adjacent to the end of the tubular section 18, averted from the distal end 19. Further, the tubular extension 24 is arranged coaxially with the tubular section 18, to form, in combination with the tubular section 18, a coherent (or: continuous) inner channel 25 ranging from the distal end 19 to the end of the tubular extension 24 averted from the distal end 19. The inner channel 25 is configured for accommodating and guiding therein the optical fiber 15, as shown for example in FIG. 3.

As may be inferred from FIG. 3, the tubular extension 24 is provided and configured for being inserted and mounted in the recess 13 of the mounting head 10. An inner shoulder 26 may be provided at the inner wall in the transitional area between the tubular section 18 and the tubular extension 24. The inner shoulder 26 may define a conical tapering of the inner diameter, wherein a further opening angle 27 of the conically tapered inner surfaces of the inner shoulder 26 may be in the range of 115 - 120 degrees, in particular 118 degrees. Such a shoulder 26 may be beneficial with regard to inserting the optical fiber 15 in the course of assembling the handheld device 1.

The tubular section 18, including the distal end 19, and optionally also the tubular extension 24, are preferably made from titanium or a titanium alloy.

Some further aspects that have been proven advantageous for generating the plasma, and optionally the pressure waves or shockwaves in connection with plasma formation, in particular suitable for treating Glaucoma as described further above, are given below.

According to exemplary embodiments, it has been proven advantageous that the outer diameter D2 of the tubular section 18, in particular of the plasma chamber 16, is in the range between 0.6 mm and 1.0 mm, for example 0.8 mm. Further, the inner diameter D1 of the tubular section 18, in particular of the plasma chamber 16, may advantageously lie in the range from 0.5 to 0.7 mm, for example 0.6 mm. A ratio between the outer diameter and the inner diameter measured in radial direction relative to the longitudinal axis L may for example be 4:3.

In embodiments, it has been proven advantageous that the average material thickness t measured in axial direction, i.e. parallel to the longitudinal axis L, at the distal end 19 is at least twice the material thickness of the tubular section 18 and/or plasma chamber 16 measured in radial direction thereof. The material thickness t may for example be about 0.2 mm

The above discussion reveals that the suggested device that enables plasma formation in an inner plasma chamber, and discharge of at least a part of the plasma out of the plasma chamber for tissue treating is suitable for solving the underlying problem.

### Reference Signs

- 1: handheld device
- 2: handpiece
- 3: supply line
- 4: connector piece
- 5: tool tip
- 6: routing element
- 7: shaft
- 8: tool tip
- 9: tubular tip
- 10: mounting head
- 11: coupler section
- 12: retainer section
- 13: recess
- 14: bore
- 15: optical fiber
- 16: plasma chamber
- 17: tip end
- 18: tubular section
- 19: distal end
- 20: central axial aperture
- 21: light injection section
- 22: inner axial face
- 23: opening angle
- 24: tubular extension
- 25: inner channel
- 26: inner shoulder
- 27: further opening angle
- 28: laser light

- a: axial length of the plasma chamber
- d: cross section of the aperture
- D1: inner diameter of the tubular section
- D2: outer diameter of the tubular section
- L1: axial length
- L2: length
- L: longitudinal axis
- t: wall thickness at the tip end

## Claims

1. Device for (1) the treatment of biological tissue, in particular for plasma treatment of biological tissue, of for example a human organism, such as for example the trabecular meshwork of the human eye, the device (1) comprising
a hollow needle-like tubular section (18) with a distal end (19) comprising a distal, central axial aperture (20), and
a light guide (15) guided in the tubular section (18),
wherein the tubular section (18) comprises subsequent to the distal end (19) an inner plasma chamber (16) configured for generating therein a light-induced plasma,
wherein the distal end (19) is configured for use in applying a light-induced plasma to the tissue by inserting the needle-like tubular section (18) into the tissue with the distal end (19) ahead such that the distal end (19) is placed at the tissue to be treated with plasma discharged from the plasma chamber (16) through the central, axial aperture (20),
wherein the plasma chamber (16) is communicatively coupled with the distal, central axial aperture (20) in the distal end (19), and adjoins, within the tubular section (18), a light injection section (21) for injecting laser light (28), in particular laser light pulses, supplied by a laser light source to the light injection section (21), into the plasma chamber (16) for plasma generation, wherein
the light injection section (21) includes, as an axial end face facing the plasma chamber (16), a light exit face (21) of the light guide (15), and the distal end (19) comprises an inner axial face (22) that is oriented towards the plasma chamber (16), the light guide (15) arranged adjacent to the plasma chamber (16) such that the light exit surface (21) faces the inner axial face (22), wherein
the inner axial face (22) acts as a laser absorption target for absorbing a part of the laser light (28) emitted from the light injection section (21), and wherein
the light guide (15) is configured and arranged such that at least a portion of the light exiting the light exit surface (21) impinges on the inner axial face (22) to generate within the plasma chamber (16) a plasma in which the volumetric material associated with the inner axial face (11) acts as the light absorption target for plasma formation.

2. Device (1) according to Claim 1, wherein the aperture (20), the plasma chamber (15), and the light injection section (21) are arranged coaxially with each other, in particular with the longitudinal axis (L) of the tubular section (18).

3. Device (1) according to Claim 1 or 2, wherein the cross section of the aperture (20) measured perpendicular to the longitudinal axis (L) of the plasma chamber (16) or of the tubular section (18) is in the range from 0.05 mm to 0.5 mm, preferably 0.05 mm to 0.3 mm.

4. Device (1) according to at least one of Claims 1 to 3, wherein the distal end (19) of the tubular section (18) is dome-shaped, and optionally has an outer rounded tip.

5. Device (1) according to at least one of Claims 1 to 4, wherein the inner axial face (22) surrounds the axial aperture (20), and optionally has an annular ring shape.

6. Device (1) according to Claim 5, wherein the inner axial face (22) is, in axial direction towards the aperture (20), conically tapered, wherein an opening angle (23) of the conically tapered axial face (22) optionally is in the range from 115 to 120 degrees, preferably 118 degrees.

7. Device (1) according to at least one of Claims 1 to 6, wherein the outer diameter (D2) of the tubular section (18), in particular or at least in the region of the plasma chamber, is in the range between 0.6 mm and 1.0 mm, preferably 0.8 mm, and wherein the inner diameter (D1) of the tubular section (18), in particular or at least in the region of the plasma chamber, is in the range between 0.5 to 0.7 mm, preferably 0.6 mm; wherein a ratio between the outer diameter (D2) and the inner diameter (D1) preferably is 4:3.

8. Device (1) according to at least one of Claims 1 to 7, wherein the wall thickness (t) of the tubular section (18) at the distal end (19) measured parallel to the longitudinal axis (L) of the tubular section (18) is in the range from 0.18 mm to 0.22 mm, preferably 0.2 mm.

9. Device (1) according to at least one of Claims 1 to 8, wherein the axial length (a) of the plasma chamber (16) measured parallel to the longitudinal central axis (L) of the tubular section (18) is at least as large as the inner diameter (D1) of the tubular section (18), preferably at least by a factor of 1.5, in particular a factor of about 1.66, larger than the inner diameter (D1) of the tubular section (18), and/or wherein the axial length (a) of the plasma chamber (16) is in the range from 0.5 mm to 1.2 mm, in particular 0.9 mm to 1.1 mm.

10. Device (1) according to at least one of Claims 1 to 9, wherein the light injection section (21) has a numerical aperture in the range from 0.2 to 0.24, in particular 0.22.

11. Device (1) according to at least one of Claims 1 to 10, wherein the light guide (15) is guided in and arranged coaxially within the tubular section (18), the plasma chamber (16) and/or the aperture (20), wherein, optionally, the axial end face (21), in particular the light exit face (21), is oriented towards the aperture (20), and wherein the light guide (15) is disposed within the tubular section such that the optical axis of the light guide (15) is coaxial with the tubular section (18), the plasma chamber (16) and/or the aperture (20), and wherein, further optionally, the light guide (15), the light exit face (21), and/or the fiber optic has a numerical aperture in the range from 0.2 to 0.24, in particular 0.22.

12. Device (1) according to at least one of Claims 1 to 11, further comprising a tubular extension (24) arranged immediately adjacent to the end of the tubular section (18) averted from the distal end (19), wherein the tubular extension (24) is arranged coaxially with the tubular section (18), and wherein the tubular extension (24) is configured for being inserted and mounted in a recess of a mounting head (10) for mounting to a handpiece (2) for manual handling of the device (1), the device (1) optionally further comprising the handpiece (2), wherein the mounting head (10) is optionally fastened to one end of the handpiece (2), and wherein a flexible duct (3) for guiding therein a light guide (15) , or when dependent from claim 11 the light guide (15), in particular the optical fiber (15), is optionally attached to the other end of the handpiece (2).

13. Device (1) according to at least one of Claims 1 to 12, wherein the light guide (15), in particular the optical fiber (15), is fastened within or at the hollow tubular section (18), the tubular extension (24), the handpiece (2) and/or the flexible duct (3) by means of at least one adhesive bond.

14. Device (1) according to at least one of Claims 1 to 13, wherein the tubular section (18), including the distal end (19), and optionally also the tubular extension (24), are made from titanium or a titanium alloy.

15. Device (1) according to at least one of Claims 1 to 14, further comprising the laser light source, wherein the laser light source is optionally a Q-switched laser, in particular a Q-switched Nd:YAG laser.

16. A device according to claim 15, wherein the laser light source is configured for generating a nanosecond laser pulse, preferably with a pulse width of 8 ns ± 3 ns, and/or with a pulse energy in the range from 6 mJ to 10 mJ.

## Patentansprüche

1. Vorrichtung (1) zur Behandlung von biologischem Gewebe, insbesondere zur Plasmabehandlung von biologischem Gewebe, zum Beispiel eines menschlichen Organismus, wie zum Beispiel das Trabekelwerk des menschlichen Auges, wobei die Vorrichtung (1) umfasst
einen hohlen, nadelartigen rohrförmigen Abschnitt (18) mit einem distalen Ende (19), das eine distale, zentrale axiale Blende (20) aufweist, und
einen Lichtleiter (15), der in dem rohrförmigen Abschnitt (18) geführt wird,
wobei der rohrförmige Abschnitt (18) im Anschluss an das distale Ende (19) eine innere Plasmakammer (16) umfasst, die so konfiguriert ist, dass darin ein lichtinduziertes Plasma erzeugt wird,
wobei das distale Ende (19) zur Verwendung beim Aufbringen eines lichtinduzierten Plasmas auf das Gewebe konfiguriert ist, indem der nadelartige rohrförmige Abschnitt (18) mit dem distalen Ende (19) voraus in das Gewebe eingeführt wird, so dass das distale Ende (19) an dem mit Plasma, das von der Plasmakammer (16) durch die zentrale axiale Blende (20) abgegeben wird, zu behandelnden Gewebe platziert wird,
wobei die Plasmakammer (16) mit der distalen, zentralen axialen Blende (20) im distalen Ende (19) kommunikativ gekoppelt ist und sich innerhalb des rohrförmigen Abschnitts (18) ein Lichteinleitungsabschnitt (21) zum Einleiten von Laserlicht (28), insbesondere von Laserlichtimpulsen, die von einer Laserlichtquelle dem Lichteinleitungsabschnitt (21) zugeführt werden, in die Plasmakammer (16) zur Plasmaerzeugung anschließt,
wobei der Lichteinleitungsabschnitt (21) als axiale, der Plasmakammer (16) zugewandte Stirnfläche eine Lichtaustrittsfläche (21) des Lichtleiters (15) aufweist und das distale Ende (19) eine innere axiale Fläche (22) aufweist, die der Plasmakammer (16) zugewandt ist, wobei der Lichtleiter (15) neben der Plasmakammer (16) so angeordnet ist, dass die Lichtaustrittsfläche (21) der inneren axialen Fläche (22) zugewandt ist,
wobei die innere axiale Fläche (22) als Laserabsorptionsziel zum Absorbieren eines Teils des von dem Lichteinleitungsabschnitt (21) emittierten Laserlichts (28) dient, und
wobei der Lichtleiter (15) so konfiguriert und angeordnet ist, dass mindestens ein Teil des aus der Lichtaustrittsfläche (21) austretenden Lichts auf die innere axiale Fläche (22) auftrifft, um in der Plasmakammer (16) ein Plasma zu erzeugen, in dem das der inneren axialen Fläche (11) zugeordnete volumetrische Material als Lichtabsorptionsziel für die Plasmabildung wirkt.

2. Vorrichtung (1) nach Anspruch 1, wobei die Blende (20), die Plasmakammer (15) und der Lichteinleitungsabschnitt (21) koaxial zueinander, insbesondere zur Längsachse (L) des rohrförmigen Abschnitts (18), angeordnet sind.

3. Vorrichtung (1) nach Anspruch 1 oder 2, wobei der senkrecht zur Längsachse (L) der Plasmakammer (16) oder des rohrförmigen Abschnitts (18) gemessene Querschnitt der Blende (20) im Bereich von 0,05 mm bis 0,5 mm, vorzugsweise 0,05 mm bis 0,3 mm liegt.

4. Vorrichtung (1) nach mindestens einem der Ansprüche 1 bis 3, wobei das distale Ende (19) des rohrförmigen Abschnitts (18) kuppelförmig ist und optional eine äußere abgerundete Spitze aufweist.

5. Vorrichtung (1) nach mindestens einem der Ansprüche 1 bis 4, wobei die innere axiale Fläche (22) die axiale Blende (20) umgibt und optional eine Ringform aufweist.

6. Vorrichtung (1) nach Anspruch 5, wobei die innere axiale Fläche (22) in axialer Richtung zur Blende (20) hin konisch verjüngt ist, wobei ein Öffnungswinkel (23) der konisch verjüngten axialen Fläche (22) wahlweise im Bereich von 115 bis 120 Grad, vorzugsweise 118 Grad, liegt.

7. Vorrichtung (1) nach mindestens einem der Ansprüche 1 bis 6, wobei der Außendurchmesser (D2) des rohrförmigen Abschnitts (18), insbesondere oder zumindest im Bereich der Plasmakammer, im Bereich zwischen 0,6 mm und 1,0 mm, vorzugsweise 0.8 mm liegt, und wobei der Innendurchmesser (D1) des rohrförmigen Abschnitts (18), insbesondere oder zumindest im Bereich der Plasmakammer, im Bereich zwischen 0,5 bis 0,7 mm, vorzugsweise 0,6 mm liegt; wobei ein Verhältnis zwischen dem Außendurchmesser (D2) und dem Innendurchmesser (D1) vorzugsweise 4:3 beträgt.

8. Vorrichtung (1) nach mindestens einem der Ansprüche 1 bis 7, wobei die Wandstärke (t) des rohrförmigen Abschnitts (18) am distalen Ende (19), gemessen parallel zur Längsachse (L) des rohrförmigen Abschnitts (18), im Bereich von 0,18 mm bis 0,22 mm, vorzugsweise 0,2 mm, liegt.

9. Vorrichtung (1) nach mindestens einem der Ansprüche 1 bis 8, wobei die axiale Länge (a) der Plasmakammer (16) gemessen parallel zur Längsachse (L) des rohrförmigen Abschnitts (18) mindestens so groß ist wie der Innendurchmesser (D1) des rohrförmigen Abschnitts (18), vorzugsweise mindestens um den Faktor 1,5, insbesondere um einen Faktor von etwa 1,66, größer ist als der Innendurchmesser (D1) des rohrförmigen Abschnitts (18), und/oder wobei die axiale Länge (a) der Plasmakammer (16) im Bereich von 0,5 mm bis 1,2 mm, insbesondere 0,9 mm bis 1,1 mm liegt.

10. Vorrichtung (1) nach mindestens einem der Ansprüche 1 bis 9, wobei der Lichteinleitungsabschnitt (21) eine numerische Blende im Bereich von 0,2 bis 0,24, insbesondere 0,22, aufweist.

11. Vorrichtung (1) nach mindestens einem der Ansprüche 1 bis 10, wobei der Lichtleiter (15) in dem rohrförmigen Abschnitt (18), der Plasmakammer (16) und/oder der Blende (20) geführt und koaxial zu dieser angeordnet ist, wobei gegebenenfalls die axiale Stirnfläche (21), insbesondere die Lichtaustrittsfläche (21), zur Blende (20) hin ausgerichtet ist, und wobei der Lichtleiter (15) innerhalb des rohrförmigen Abschnitts so angeordnet ist, dass die optische Achse des Lichtleiters (15) koaxial mit dem rohrförmigen Abschnitt (18), der Plasmakammer (16) und/oder der Blende (20) ist, und wobei ferner optional der Lichtleiter (15), die Lichtaustrittsfläche (21) und/oder die Faseroptik eine numerische Blende im Bereich von 0.2 bis 0,24, insbesondere 0,22, aufweist.

12. Vorrichtung (1) nach mindestens einem der Ansprüche 1 bis 11, ferner umfassend eine röhrenförmige Erweiterung (24), die unmittelbar benachbart zu dem dem distalen Ende (19) abgewandten Ende des rohrförmigen Abschnitts (18) angeordnet ist, wobei die röhrenförmige Erweiterung (24) koaxial zu dem rohrförmigen Abschnitt (18) angeordnet ist, und wobei die röhrenförmige Erweiterung (24) zum Einsetzen und Montieren in einer Aussparung eines Montagekopfes (10) zum Montieren an einem Handstück (2) zur manuellen Handhabung der Vorrichtung (1) ausgebildet ist, die Vorrichtung (1) optional weiterhin das Handstück (2) umfasst, wobei der Montagekopf (10) optional an einem Ende des Handstücks (2) befestigt ist, und wobei ein flexibler Kanal (3) zum Führen eines Lichtleiters (15) darin, oder in Abhängigkeit von Anspruch 11 der Lichtleiter (15), insbesondere die optische Faser (15), optional an dem anderen Ende des Handstücks (2) befestigt ist.

13. Vorrichtung (1) nach mindestens einem der Ansprüche 1 bis 12, wobei der Lichtleiter (15), insbesondere die optische Faser (15), innerhalb oder an dem hohlen rohrförmigen Abschnitt (18), der röhrenförmigen Erweiterung (24), dem Handstück (2) und/oder dem flexiblen Kanal (3) mittels mindestens einer Klebeverbindung befestigt ist.

14. Vorrichtung (1) nach mindestens einem der Ansprüche 1 bis 13, wobei der rohrförmige Abschnitt (18), einschließlich des distalen Endes (19), und gegebenenfalls auch die röhrenförmige Erweiterung (24) aus Titan oder einer Titanlegierung hergestellt sind.

15. Vorrichtung (1) nach mindestens einem der Ansprüche 1 bis 14, ferner umfassend die Laserlichtquelle, wobei die Laserlichtquelle optional ein gütegeschalteter Laser, insbesondere ein gütegeschalteter Nd:YAG-Laser ist.

16. Vorrichtung nach Anspruch 15, wobei die Laserlichtquelle zur Erzeugung eines Nanosekunden-Laserpulses, vorzugsweise mit einer Pulsbreite von 8 ns ± 3 ns, und/oder mit einer Pulsenergie im Bereich von 6 mJ bis 10 mJ konfiguriert ist.

## Revendications

1. Dispositif pour (1) le traitement de tissus biologiques, en particulier pour le traitement par plasma de tissus biologiques, par exemple d'un organisme humain, comme par exemple le réseau trabéculaire de l'œil humain, le dispositif (1) comprenant
une section tubulaire creuse en forme d'aiguille (18) avec une extrémité distale (19) comprenant une ouverture axiale centrale distale (20), et
un guide de lumière (15) guidé dans la section tubulaire (18),
la section tubulaire (18) comprend, après l'extrémité distale (19), une chambre à plasma interne (16) configurée pour générer un *plasma* induit par la lumière,
dans lequel l'extrémité distale (19) est configurée pour être utilisée dans l'application d'un plasma induit par la lumière au tissu en insérant la section tubulaire en forme d'aiguille (18) dans le tissu avec l'extrémité distale (19) en avant de telle sorte que l'extrémité distale (19) est placée sur le tissu à traiter avec le plasma déchargé de la chambre à plasma (16) à travers l'ouverture centrale, axiale (20),
dans laquelle la chambre à plasma (16) est couplée de manière communicative avec l'ouverture axiale centrale distale (20) de l'extrémité distale (19) et jouxte, à l'intérieur de la section tubulaire (18), une section d'injection de lumière (21) pour injecter de la lumière laser (28), en particulier des impulsions de lumière laser, fournie par une source de lumière laser à la section d'injection de lumière (21), dans la chambre à plasma (16) pour la génération de plasma, dans laquelle
la section d'injection de lumière (21) comprend, en tant que face d'extrémité axiale orientée vers la chambre à plasma (16), une face de sortie de lumière (21) du guide de lumière (15), et l'extrémité distale (19) comprend une face axiale intérieure (22) orientée vers la chambre à plasma (16), le guide de lumière (15) étant disposé à côté de la chambre à plasma (16) de telle sorte que la surface de sortie de lumière (21) soit orientée vers la face axiale intérieure (22), dans lequel
la face axiale intérieure (22) agit comme une cible d'absorption laser pour absorber une partie de la lumière laser (28) émise par la section d'injection de lumière (21), et dans laquelle
le guide de lumière (15) est configuré et disposé de manière à ce qu'au moins une partie de la lumière sortant de la surface de sortie de la lumière (21) frappe la face axiale intérieure (22) pour générer dans la chambre à plasma (16) un plasma dans lequel le matériau volumétrique associé à la face axiale intérieure (11) agit comme cible d'absorption de la lumière pour la formation du plasma.

2. Dispositif (1) selon la revendication 1, dans lequel l'ouverture (20), la chambre à plasma (15) et la section d'injection de lumière (21) sont disposées coaxialement les unes par rapport aux autres, en particulier par rapport à l'axe longitudinal (L) de la section tubulaire (18).

3. Dispositif (1) selon la revendication 1 ou 2, dans lequel la section transversale de l'ouverture (20) mesurée perpendiculairement à l'axe longitudinal (L) de la chambre à plasma (16) ou de la section tubulaire (18) est comprise entre 0,05 mm et 0,5 mm, de préférence entre 0,05 mm et 0,3 mm.

4. Dispositif (1) selon au moins l'une des revendications 1 à 3, dans lequel l'extrémité distale (19) de la section tubulaire (18) est en forme de dôme et présente éventuellement une pointe arrondie extérieure.

5. Dispositif (1) selon au moins l'une des revendications 1 à 4, dans lequel la face axiale intérieure (22) entoure l'ouverture axiale (20) et présente éventuellement une forme d'anneau annulaire.

6. Dispositif (1) selon la revendication 5, dans lequel la face axiale intérieure (22) est conique dans la direction axiale vers l'ouverture (20), dans lequel un angle d'ouverture (23) de la face axiale conique (22) est éventuellement compris entre 115 et 120 degrés, de préférence 118 degrés.

7. Dispositif (1) selon au moins l'une des revendications 1 à 6, dans lequel le diamètre extérieur (D2) de la section tubulaire (18), en particulier ou au moins dans la région de la chambre à plasma, est compris entre 0,6 mm et 1,0 mm, de préférence 0,8 mm, et dans lequel le diamètre intérieur (D1) de la section tubulaire (18), en particulier ou au moins dans la région de la chambre à plasma, est compris entre 0,5 et 0,7 mm.8 mm, et dans lequel le diamètre intérieur (D1) de la section tubulaire (18), en particulier ou au moins dans la région de la chambre à plasma , est compris entre 0,5 et 0,7 mm, de préférence 0,6 mm ; le rapport entre le diamètre extérieur (D2) et le diamètre intérieur (D1) est de préférence de 4:3.

8. Dispositif (1) selon au moins l'une des revendications 1 à 7, dans lequel l'épaisseur de la paroi (t) de la section tubulaire (18) à l'extrémité distale (19), mesurée parallèlement à l'axe longitudinal (L) de la section tubulaire (18), est comprise entre 0,18 mm et 0,22 mm, de préférence 0,2 mm.

9. Dispositif (1) selon au moins l'une des revendications 1 à 8, dans lequel la longueur axiale (a) de la chambre à plasma (16) mesurée parallèlement à l'axe central longitudinal (L) de la section tubulaire (18) est au moins aussi grande que le diamètre intérieur (D1) de la section tubulaire (18), de préférence au moins d'un facteur de 1.5, en particulier un facteur d'environ 1,66, plus grand que le diamètre intérieur (D1) de la section tubulaire (18), et/ou dans lequel la longueur axiale (a) de la chambre à plasma (16) est comprise entre 0,5 mm et 1,2 mm, en particulier entre 0,9 mm et 1,1 mm.

10. Dispositif (1) selon au moins l'une des revendications 1 à 9, dans lequel la section d'injection de lumière (21) présente une ouverture numérique comprise entre 0,2 et 0,24, en particulier 0,22.

11. Dispositif (1) selon au moins l'une des revendications 1 à 10, dans lequel le guide de lumière (15) est guidé et disposé coaxialement dans la section tubulaire (18), la chambre à plasma (16) et/ou l'ouverture (20), dans lequel, éventuellement, la face d'extrémité axiale (21), en particulier la face de sortie de la lumière (21), est orientée vers l'ouverture (20), et dans lequel le guide de lumière (15) est disposé dans la section tubulaire de telle sorte que l'axe optique du guide de lumière (15) est coaxial avec la section tubulaire (18), la chambre à plasma (16) et/ou l'ouverture (20), et dans lequel, en outre, le guide de lumière (15), la face de sortie de la lumière (21), et/ou la fibre optique a une ouverture numérique dans la plage de 0.2 à 0,24, en particulier 0,22.

12. Dispositif (1) selon au moins l'une des revendications 1 à 11, comprenant en outre une extension tubulaire (24) disposée immédiatement à côté de l'extrémité de la section tubulaire (18) opposée à l'extrémité distale (19), dans laquelle l'extension tubulaire (24) est disposée coaxialement avec la section tubulaire (18), et dans laquelle l'extension tubulaire (24) est configurée pour être insérée et montée dans une cavité d'une tête de montage (10) destinée à être montée sur une pièce à main (2) pour le maniement manuel du dispositif (1), le dispositif (1) comprend en outre éventuellement la pièce à main (2), dans laquelle la tête de montage (10) est éventuellement fixée à une extrémité de la pièce à main (2), et dans laquelle un conduit flexible (3) pour y faire passer un guide de lumière (15), ou lorsqu'il dépend de la revendication 11, le guide de lumière (15), en particulier la fibre optique (15), est éventuellement fixé à l'autre extrémité de la pièce à main (2).

13. Dispositif (1) selon au moins l'une des revendications 1 à 12, dans lequel le guide de lumière (15), en particulier la fibre optique (15), est fixé à l'intérieur ou au niveau de la section tubulaire creuse (18), de l'extension tubulaire (24), de la pièce à main (2) et/ou du conduit flexible (3) au moyen d'au moins une liaison adhésive.

14. Dispositif (1) selon au moins l'une des revendications 1 à 13, dans lequel la section tubulaire (18), y compris l'extrémité distale (19), et éventuellement l'extension tubulaire (24), sont en titane ou en alliage de titane.

15. Dispositif (1) selon au moins l'une des revendications 1 à 14, comprenant en outre la source de lumière laser, dans laquelle la source de lumière laser est éventuellement un laser Q-switché, en particulier un laser Nd:YAG Q-switché.

16. Dispositif selon la revendication 15, dans lequel la source de lumière laser est configurée pour générer une impulsion laser nanoseconde, de préférence avec une largeur d'impulsion de 8 ns ± 3 ns, et/ou avec une énergie d'impulsion comprise entre 6 mJ et 10 mJ.
